(19) **Europäisches Patentamt / European Patent Office / Office européen des brevets**

(11) **EP 4 768 464 A1**

(12) # EUROPEAN PATENT APPLICATION
published in accordance with Art. 153(4) EPC

(43) Date of publication:
**01.07.2026 Bulletin 2026/27**

(21) Application number: **25756106.8**

(22) Date of filing: **11.03.2025**

(51) International Patent Classification (IPC):
*C07C 37/84* (2006.01)    *C07C 37/68* (2006.01)
*C07C 37/74* (2006.01)    *C07C 37/055* (2006.01)
*C07C 39/16* (2006.01)    *C08J 11/24* (2006.01)
*C08G 64/30* (2006.01)

(52) Cooperative Patent Classification (CPC):
(C-Sets available)
**C07C 37/0555; C07C 37/74; C07C 37/84;
C07C 39/16; C08G 64/30; C08J 11/24;**
C08J 2369/00; Y02W 30/62          (Cont.)

(86) International application number:
**PCT/KR2025/003154**

(87) International publication number:
**WO 2026/095223 (07.05.2026 Gazette 2026/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.10.2024 KR 20240151994**

(71) Applicant: **LG Chem, Ltd.**
**Seoul 07336 (KR)**

(72) Inventors:
• **LEE, Jinhyeok**
  **Yuseong-gu, Daejon 34122 (KR)**
• **JEON, Byoungkue**
  **Yuseong-gu, Daejon 34122 (KR)**
• **HONG, Mooho**
  **Yuseong-gu, Daejon 34122 (KR)**
• **PARK, Tae Seung**
  **Yuseong-gu, Daejon 34122 (KR)**
• **KIM, Minju**
  **Yuseong-gu, Daejon 34122 (KR)**

(74) Representative: **Goddar, Heinz J.**
**Boehmert & Boehmert
Anwaltspartnerschaft mbB
Pettenkoferstrasse 22
80336 München (DE)**

(54) **METHOD FOR PREPARING MONOMER FOR SYNTHESIZING RECYCLED PLASTIC, MONOMER FOR SYNTHESIZING RECYCLED PLASTIC USING SAME, RECYCLED PLASTIC, AND MOLDED ARTICLE**

(57)    The present disclosure relates to a preparation method of a monomer for synthesizing recycled plastic, the method comprising the steps of: a step of recovering an aromatic diol compound obtained from the depolymerization reaction of a polycarbonate-based resin; a step of melting the aromatic diol compound; a step of crystallizing the melted aromatic diol compound; and a step of obtaining the crystallized aromatic diol compound, and a monomer for synthesizing recycled plastic, a recycled plastic and a molded product using the same.

EP 4 768 464 A1

**(Cont. next page)**

(52)  Cooperative Patent Classification (CPC): (Cont.)

C-Sets
**C07C 37/0555, C07C 39/16;**
**C07C 37/74, C07C 39/16;**
**C07C 37/84, C07C 39/16**

**Description**

[**TECHNICAL FIELD**]

CROSS-REFERENCE TO RELATED APPLICATION(S)

This application claims the benefit of Korean Patent Application No. 10-2024-0151994 filed on October 31, 2024 with the Korean Intellectual Property Office, the disclosure of which is incorporated herein by reference in its entirety.

The present disclosure relates to a preparation method of a monomer for synthesizing recycled plastic that contains a high-purity, high-yield aromatic diol compound recovered through recycling by chemical decomposition of a polycarbonate-based resin, a monomer for synthesizing recycled plastic, a recycled plastic and a molded product using the same.

[BACKGROUND ART]

Polycarbonate is a thermoplastic polymer and is a plastic having excellent characteristics such as excellent transparency, ductility, and relatively low manufacturing cost.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of polycarbonate refers to obtaining an aromatic diol compound as a monomer (e.g., bisphenol A (BPA)) through decomposition of polycarbonate, and then utilizing it again in polymerization to obtain a high-purity polycarbonate.

For such a chemical decomposition, thermal decomposition, hydrolysis, and alcohol decomposition are typically known. Among these, the most common method is alcohol decomposition using a base catalyst, but in the case of methanol decomposition, there is a problem that methanol is used which is harmful to the human body, and in the case of ethanol, there is a problem that high temperature and high pressure conditions are required and the yield is not high.

In addition, although an alcohol decomposition method using an organic catalyst is known, it is disadvantageous in terms of economics.

[DETAILED DESCRIPTION OF THE INVENTION]

[Technical Problem]

It is an object of the present invention to provide a monomer for synthesizing recycled plastic that can secure a high-purity, high-yield of an aromatic diol compound through recycling by chemical decomposition of polycarbonate-based resin.

It is another object of the present invention to provide a method for preparing the monomer for synthesizing recycled plastic, and a recycled plastic, and molded product using the method for preparing the monomer for synthesizing recycled plastic.

[Technical Solution]

In order to achieve the above object, provided herein is a preparation method of a monomer for synthesizing recycled plastic, which includes: a step of recovering an aromatic diol compound obtained from the depolymerization reaction of a polycarbonate-based resin; a step of melting the aromatic diol compound; a step of crystallizing the melted aromatic diol compound; and a step of obtaining the crystallized aromatic diol compound.

Further provided herein is a monomer for synthesizing recycled plastic, comprising an aromatic diol compound obtained by the preparation method of a monomer for synthesizing recycled plastic.

Further provided herein is a recycled plastic comprising a reaction product of the monomer for synthesizing recycled plastic and a comonomer.

Further provided herein is a molded product comprising the recycled plastic.

Below, a preparation method of a monomer for synthesizing recycled plastic, and a monomer for synthesizing recycled plastic, a recycled plastic and a molded product using the same according to specific embodiments of the present disclosure will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

The 'pH' as used herein means a hydrogen ion concentration (pH), which is a numerical value indicating the acidity and alkalinity of a material. The pH can be determined from a value expressed by taking the reciprocal of the logarithmic dissociation concentration of hydrogen ions, and is used as a measure of the strength of acids and bases of a material.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

1. Preparation Method of Monomer for Synthesizing Recycled Plastic

According to one embodiment of the present disclosure, there can be provided a preparation method of a monomer for synthesizing recycled plastic, which includes: a step of recovering an aromatic diol compound obtained from the depolymerization reaction of a polycarbonate-based resin; a step of melting the aromatic diol compound; a step of crystallizing the melted aromatic diol compound; and a step of obtaining the crystallized aromatic diol compound.

The inventors have confirmed through experiments and completed the invention by purifying the aromatic diol compound obtained in the process of recycling polycarbonate-based resin through chemical decomposition using a melt crystallization process, thereby ensuring a high-purity aromatic diol compound at a high yield with minimized impurity content, even though it was recovered through chemical decomposition recycling of polycarbonate-based resin.

In particular, during the process of melting the aromatic diol compound, there is a problem of reduced yield due to the decomposition of the aromatic diol compound or solidification of the melted aromatic diol compound. To solve this, the melting and crystallization conditions of the aromatic diol compound can be optimized to improve the purity and yield of the aromatic diol compound.

As a result, with the significant reduction of impurity content other than the main target aromatic diol compound, it has been confirmed through experiments that excellent physical properties can be achieved when synthesizing polycarbonate-based resin using this compound, thus completing the invention.

Specifically, the method for producing monomers for recycling plastic synthesis in the embodiment may include the step of recovering the aromatic diol compound obtained by the depolymerization reaction of a polycarbonate-based resin.

More specifically, the step of recovering the aromatic diol compound obtained by the depolymerization reaction of the polycarbonate-based resin may include depolymerizing the polycarbonate-based resin; adding acid to the depolymerization reaction product for neutralization; removing the water layer from the water and organic solvent layers formed in the neutralization step; and distilling the organic solvent layer to recover the aromatic diol compound.

First, in the step of depolymerizing the polycarbonate-based resin, the polycarbonate-based resin refers to both a homopolymer and a copolymer containing polycarbonate repeating units, and is a general term for reaction products obtained through polymerization or copolymerization reactions of monomers including aromatic diol compounds and carbonate precursors. A homopolymer can be synthesized when only one type of aromatic diol compound and one type of carbonate precursor are used to obtain a polycarbonate repeating unit. Additionally, a copolymer can be synthesized when more than two types of carbonate are contained by using one type of aromatic diol compound and more than two types of carbonate precursors, or by using more than two types of aromatic diol compounds and one type of carbonate precursor, or by using additional diols beyond one type of aromatic diol compound and one type of carbonate precursor. The homopolymer or copolymer may include low molecular compounds, oligomers, and polymers according to the molecular weight range.

The above-mentioned polycarbonate-based resin can be applied regardless of various forms and types, such as newly synthesized polycarbonate-based resin produced through synthesis, recycled polycarbonate-based resin produced through a recycling process, or polycarbonate-based resin waste.

During the depolymerization reaction of the polycarbonate-based resin, the depolymerization reaction can be performed under acidic, neutral, or basic conditions, and in particular, the depolymerization reaction can proceed under basic (alkaline) conditions. The type of base is not particularly limited, and examples include sodium hydroxide (NaOH) or potassium hydroxide (KOH). The base acts as a base catalyst, offering an economic advantage over organic catalysts mainly used under mild conditions. More specifically, during the depolymerization reaction of the polycarbonate-based resin, the depolymerization reaction can proceed within a pH range of more than 8 and less than 12.

During the depolymerization reaction of the polycarbonate-based resin, the reaction can proceed by reacting the base in an amount of 0.5 mol or less, or 0.4 mol or less, or 0.3 mol or less, or 0.1 mol or more, or 0.2 mol or more, or 0.1 mol to 0.5 mol, or 0.1 mol to 0.4 mol, or 0.1 mol to 0.3 mol, or 0.2 mol to 0.5 mol, or 0.2 mol to 0.4 mol, or 0.2 mol to 0.3 mol per 1 mol of polycarbonate-based resin. If the base is reacted in excess of 0.5 mol per 1 mol of polycarbonate-based resin during the depolymerization reaction, impurities increase due to the increased production of alkaline salts, reducing the purity of the target recovery material and decreasing the economic efficiency of the catalytic reaction.

Additionally, the depolymerization reaction of the polycarbonate-based resin can proceed in a solvent containing ethanol. This invention allows for the stable acquisition of high-purity bisphenol A, a monomer, by decomposing the polycarbonate-based resin in a solvent containing ethanol, and also has the advantage of additionally obtaining diethyl carbonate, a high-value-added byproduct. The content of ethanol can be 5 mol to 15 mol, or 8 mol to 13 mol per 1 mol of polycarbonate-based resin. Since ethanol has good solubility for bisphenol A, ethanol within this range must be included. If the content of ethanol is excessively reduced to less than 5 mol per 1 mol of polycarbonate-based resin, it becomes difficult for the alcohol decomposition of the polycarbonate-based resin to proceed sufficiently. On the other hand, if the content of ethanol is excessively increased to more than 15 mol per 1 mol of polycarbonate-based resin, the economic efficiency of the process may decrease due to the excessive use of alcohol.

The solvent in which the depolymerization reaction of the polycarbonate-based resin proceeds, aside from ethanol, may further include one or more organic solvents selected from the group consisting of tetrahydrofuran, toluene, methylene chloride, chloroform, dimethyl carbonate, ethyl methyl carbonate, diethyl carbonate, and dipropyl carbonate. The organic solvent may include tetrahydrofuran, toluene, methylene chloride, chloroform, dimethyl carbonate, ethyl methyl carbonate, diethyl carbonate, dipropyl carbonate, or mixtures of two or more thereof. More preferably, methylene chloride can be used as the organic solvent. When methylene chloride is used as the organic solvent mixed with ethanol, there is an advantage of improved solubility characteristics for polycarbonate, thereby enhancing reactivity.

The content of the organic solvent may be 16 moles to 20 moles, or 16 moles to 18 moles, per mole of polycarbonate-based resin. Additionally, the content of the organic solvent may be 1.5 moles to 2 moles per mole of ethanol. Within this range, there is an advantage in that the depolymerization reaction of the polymer can proceed to the necessary level by mixing the polycarbonate-based resin, ethanol, and the organic solvent.

Meanwhile, the temperature at which the depolymerization reaction of the polycarbonate-based resin is conducted is not significantly limited, but for example, it can proceed at 20°C to 100°C, or 50°C to 70°C. Moreover, the time for conducting the depolymerization reaction of the polycarbonate-based resin can range from 1 hour to 30 hours, or from 4 hours to 6 hours.

Specifically, these conditions are milder process conditions compared to conventional pressurized/high-temperature processes, and by performing agitation under these conditions, the process can be carried out in a mild manner compared to pressurized/high-temperature processes. Particularly, there is an advantage of obtaining the most efficient results in terms of reproducibility and acceptance when agitating at 50°C to 70°C for 4 to 6 hours.

In other words, the present invention can obtain high-purity aromatic diol compounds (for example, bisphenol A) under mild conditions without using an organic catalyst by adjusting the type and amount of mixed solvent and the type and content of the base catalyst, without using a pressurized/high-temperature process. Furthermore, using ethanol as the solvent, there is an advantage of obtaining diethyl carbonate as a byproduct.

Meanwhile, during the depolymerization reaction of the polycarbonate-based resin, an antioxidant can be added to the reaction solution. By adding the antioxidant, the aromatic diol compound recovered through chemical decomposition recycling of the polycarbonate-based resin can satisfy a low color coordinate b* value equivalent to commercially available reagents used for PC polymerization or sale.

The specific examples of the antioxidant are not particularly limited, and various antioxidants widely used in the conventional technical field can be applied without restriction. For instance, sodium hydrosulfite, sodium sulfite, erythorbic acid, dibutylhydroxytoluene, butylated hydroxyanisole, $\alpha$-tocopherol, tocopheryl acetate, L-ascorbic acid and its salts, L-ascorbyl palmitate, L-ascorbyl stearate, triamyl gallate, propyl gallate, or ethylenediamine tetraacetic acid disodium (EDTA), sodium pyrophosphate, sodium metaphosphate, or mixtures of two or more thereof can be mentioned.

The specific amount of the antioxidant added is also not particularly limited, but for example, it can be added in an amount of 0.1% by weight to 5% by weight, or 0.1% by weight to 2% by weight, or 0.5% by weight to 2% by weight based on the total weight of the reaction solution, to a level that does not affect the properties of the monomer for recycling plastic synthesis.

Additionally, during the depolymerization reaction of the polycarbonate-based resin, the depolymerization reaction can be carried out in a nitrogen atmosphere.

Meanwhile, in the step of neutralizing the depolymerization reaction product by adding an acid, a strong acid can be used, for example, hydrochloric acid (HCl).

In the step of neutralizing the depolymerization reaction product by adding an acid, the salt of the aromatic diol compound contained in the depolymerization reaction product can be converted into an aromatic diol compound. As the depolymerization reaction proceeds under basic conditions, the resulting aromatic diol compound exists in the form of a salt due to its reaction with the base, thus having hydrophilicity. Therefore, by adding an acid, the salt of the aromatic diol compound contained in the depolymerization reaction product can be converted into an aromatic diol compound to induce hydrophobicity.

Thus, after proceeding with the step of neutralizing the depolymerization reaction product by adding an acid, layers can be formed that are separated into a water layer containing impurities and an organic solvent layer containing the aromatic diol compound and carbonate precursor. Since the aromatic diol compound and the carbonate precursor are hydrophobic, they can be included in the organic solvent layer among water and organic solvents, and various water-soluble impurities can be included in the aqueous layer. Accordingly, the main product, the aromatic diol compound, and impurities can be easily separated through a simple process of changing the pH.

Meanwhile, in the step of neutralizing the depolymerization reaction product by adding an acid, a step of adding water can be further included. Through this, layers can be formed that are separated into an aqueous layer containing impurities and an organic solvent layer containing the aromatic diol compound and carbonate precursor, as will be described later in the impurity removal step.

The order of the process of adding acid and the process of adding water is not particularly limited, and it is possible to add water after adding acid, add acid after adding water, or add water and acid simultaneously.

Meanwhile, in the step of removing the water layer from the water layer and organic solvent layer formed in the neutralization step, the water layer containing impurities can be separated and removed from the layers divided into the water layer containing impurities and the organic solvent layer containing aromatic diol compounds and carbonate precursors. The impurities are hydrophilic substances and may include, for example, salt compounds, ionic compounds, acid compounds, and the like.

The specific separation conditions for removing the water layer from the organic solvent layer are not particularly limited, and various known purification technologies can be applied without restriction regarding specific separation equipment and methods. However, as an example, a drain device may be used.

Meanwhile, in the step of distilling the organic solvent layer to recover the aromatic diol compound, the carbonate precursor can be separated from the depolymerization reaction product. The separated carbonate precursor may include diethyl carbonate.

The separated carbonate precursor can be recycled without a separate separation and purification process, or it can be recycled through conventional extraction, adsorption, drying, and other separation and purification processes as necessary. The specific purification conditions are not particularly limited, and various known purification technologies can be applied without restriction regarding specific purification equipment and methods.

More specifically, the step of distilling the organic solvent layer to recover the aromatic diol compound may include a multi-stage Distillation under reduced pressure step that distills the organic solvent layer under reduced pressure in three or more stages. In this step, by performing multi-stage Distillation under reduced pressure divided into three or more stages, namely, the first, second, and third stages, impurities other than the aromatic diol compound can be effectively removed to ensure high purity.

The specific examples of the multi-stage Distillation under reduced pressure step are not particularly limited, but as an example, the first Distillation under reduced pressure step can be performed by depressurized distilling the organic solvent layer under a pressure of 0.45 MPa or more and a temperature of 160°C or more to a pressure of 0.12 MPa to 0.45 MPa and a temperature of 48°C to 160°C. The residual solution from the first Distillation under reduced pressure step is then subjected to a second Distillation under reduced pressure step by pressurizing it to a pressure of 0.45 MPa or more and a temperature of 125°C or more, followed by depressurized distilling at a pressure of 0.009 MPa to 0.45 MPa and a temperature of 42°C to 160°C. The residual solution from the second Distillation under reduced pressure step is then subjected to a third Distillation under reduced pressure step by reducing the pressure to 0.0005 MPa or more and a temperature of 155°C or more, followed by depressurized distilling at a pressure of 0.0003 MPa to 0.0005 MPa and a temperature of 42°C to 87°C.

In the first Distillation under reduced pressure step, the organic solvent layer can be distilled under reduced pressure at a pressure of 0.45 MPa or more and a temperature of 160°C or more to a pressure of 0.12 MPa to 0.45 MPa and a temperature of 48°C to 160°C. Through this process, most of the organic solvent, ethanol, and water contained in the organic solvent layer can be separated and removed by Distillation under reduced pressure. Although the examples of Distillation under reduced pressure conditions are not particularly limited, as a specific example, the organic solvent layer can be introduced into the distillation column at a pressure of 0.45 MPa and a temperature of 160°C, and distilled by reducing the pressure to 0.12 MPa and a temperature of 48°C to 160°C.

In the second distillation step, the residual solution from the first Distillation under reduced pressure step can be subjected by pressurizing it to a pressure of 0.45 MPa or more and a temperature of 125°C or more, followed by Distillation under reduced pressure at a pressure of 0.009 MPa and a temperature of 42°C to 160°C. Through this process, the organic solvent, mainly DEC, contained in the residual solution from the first distillation step can be separated and removed by Distillation under reduced pressure. Although the examples of Distillation under reduced pressure conditions are not particularly limited, as a specific example, the residual solution from the first distillation step can be pressurized to a pressure of 0.45 MPa and a temperature of 125°C, and distilled by reducing the pressure to 0.009 to 0.45 MPa and a temperature of 42°C to 160°C.

In the third distillation step, the residual solution from the second Distillation under reduced pressure step can be subjected by reducing the pressure to 0.0005 MPa or more and a temperature of 155°C or more, followed by Distillation under reduced pressure at a pressure of 0.0003 MPa to 0.0005 MPa and a temperature of 42°C to 87°C. Through this process, the organic solvents and phenolic compounds contained in the residual solution from the second distillation step can be separated and removed by Distillation under reduced pressure. Although the examples of Distillation under reduced pressure conditions are not particularly limited, as a specific example, the residual solution from the second distillation step can be subjected by reducing the pressure to 0.0005 MPa and a temperature of 155°C, and further reduced to a pressure of 0.0003 MPa and a temperature of 42°C to 162°C for distillation under reduced pressure.

Meanwhile, before the step of recovering the aromatic diol compound obtained from the depolymerization reaction of the polycarbonate-based resin, a pretreatment step of passing the polycarbonate-based resin through a filter with a pore size of 0.1 μm or less may be further included. Through this pretreatment step, various impurities can be filtered and removed.

The polycarbonate-based resin can be passed through the filter in a solution state dissolved in an organic solvent, and the organic solvent may include tetrahydrofuran, toluene, methylene chloride, chloroform, dimethyl carbonate, ethyl methyl carbonate, diethyl carbonate, dipropyl carbonate, or a mixture of two or more thereof.

The pretreatment step is carried out under temperature conditions where the solvent does not volatilize, and a filter with a pore size of 0.3 μm or less can be used to remove fine impurities of 0.3 μm or less. The type of filter can be applied without limitation, such as a type using a filter aid or a cartridge type.

Meanwhile, the method for producing monomers for recycled plastic synthesis in this embodiment may include the step of melting the aromatic diol compound; and the step of crystallizing the melted aromatic diol compound.

The product obtained through the step of recovering the aromatic diol compound obtained from the depolymerization reaction of the polycarbonate-based resin is mostly bisphenol A, with a small amount of impurities such as aromatic diol compounds or bisphenol dimers. The melt crystallization purification method is a method of purification using the difference in solubility according to temperature between the above impurities dissolved in the melted bisphenol A.

Specifically, the step of melting the aromatic diol compound may involve heating the aromatic diol compound at a temperature of 190 °C or less, or 180 °C to 190 °C. The specific example of the time for conducting the heating is not particularly limited, but for example, it can be heated for 9 hours or less, or for 6 to 8 hours.

Since aromatic diol compounds tend to decompose at 160 °C or higher, they can be melted by temporarily heating to a temperature of 160 °C or higher for a very short time. Therefore, if the aromatic diol compound is excessively heated at a temperature of 190 °C or higher for too long, there may be a problem of reduced yield due to the decomposition of the aromatic diol compound.

Meanwhile, before the step of crystallizing the melted aromatic diol compound, a step of melt purifying the melted aromatic diol compound at a temperature of 130 °C to 180 °C for 2 to 6 hours, or for 2 to 3 hours, may be further included. By additionally melt purifying the melted aromatic diol compound before the step of crystallizing the melted aromatic diol compound in this way, impurities other than the aromatic diol compound can be effectively removed to ensure high purity.

More specifically, the step of melt purifying may include the step of cooling the melted aromatic diol compound to form aromatic diol compound crystals; recovering the purified aromatic diol compound crystals obtained by partially melting the aromatic diol compound crystals; and melting the purified aromatic diol compound crystals.

The step of cooling the molten aromatic diol compound to form aromatic diol compound crystals may involve cooling the molten aromatic diol compound to a temperature below 158°C. Since bisphenol A, a specific example of the aromatic diol compound, has a melting point of 158°C, cooling it to below 158°C can cause the aromatic diol compound, which was in a liquid state, to transition to a solid state.

The step of recovering the purified aromatic diol compound crystals obtained by partially melting the aromatic diol compound crystals can involve raising the temperature of the aromatic diol compound crystals to partially melt them so they have an intermediate character between solid and liquid. The purified aromatic diol crystals recovered here can achieve a higher purity as impurities are further refined.

Meanwhile, the purified aromatic diol compound crystals are transported in a molten state for smooth movement within equipment.

As described above, since aromatic diol compounds tend to decompose at temperatures above 160°C, it is desirable to temporarily heat them to temperatures above 160°C for a very short time for partial melting or melting of the aromatic diol compound. If the aromatic diol compound is heated excessively at temperatures above 160°C for too long, there may be a problem of reduced yield due to the decomposition of the aromatic diol compound.

Meanwhile, the melting purification step can be repeated 2 to 5 cycles, or 3 to 4 cycles. One cycle of the melting purification step proceeds in the order of cooling - sweating - melting, and the ratio of impurities removed in each cycle varies depending on the type of impurity. The starting temperature and cooling temperature of each cycle differ slightly, and it is essential to control the total cycle time to 2 to 3 hours or less to maximally prevent the quality degradation of bisphenol A. As each cycle progresses, the purity of bisphenol A increases, and other impurities such as aromatic diol compounds are drained and discharged to the bottom of the crystallizer. To improve yield, the discharged drain is reused with the feed of the previous cycle or partially recovered as bisphenol A through a static type crystallizer.

Although specific methods and equipment for performing the melting purification step are not limited, for example, purification can be carried out by one-stage fractional melt crystallization or multi-stage fractional melt crystallization in a falling film dynamic crystallizer. In this invention, results were obtained by utilizing Sulzer's technology, which has excellent technical capability in the field of melt crystallization processes.

The composition in the form of a melt is usually subjected to multi-stage fractional melt crystallization. The temperature is gradually lowered until it is below the melting point of the desired substance [in the case of bisphenol-A, MP 156.7°C]. In some cases, the composition must be heated above the melting point of the desired substance and then rapidly lowered below the freezing point. Clearly, this particular process is advantageous for separating impurities from the desired component of the composition. Ideally, the desired component crystallizes on the surface of the vessel holding the molten composition. The theory of fractional melt crystallization is to preferably crystallize the desired component from the melt while leaving the undesired impurities in their liquid state or capturing them to a limited extent within the crystalline medium. In multi-stage fractional melt crystallization, the purity of the desired crystalline component improves as it passes through the crystallization phase, sweating phase, and full melting phase in each successive stage. A preferred device for performing fractional melt crystallization is the Sulzer melt crystallization apparatus, specifically, a falling film dynamic crystallizer.

Meanwhile, the step of crystallizing the melted aromatic diol compound may involve cooling the melted aromatic diol compound to a temperature below 158°C. Since bisphenol A, a specific example of the aromatic diol compound, has a melting point of 158°C, cooling it to a temperature below 158°C can cause the aromatic diol compound, which was in a liquid state, to undergo a phase transition into a solid.

Meanwhile, the method for manufacturing monomers for recycled plastic synthesis in this embodiment may include the step of obtaining the crystallized aromatic diol compound.

In the step of obtaining the crystallized aromatic diol compound, if necessary, a process for removing residual impurities through filtration or adsorption of the crystallized aromatic diol compound may be carried out.

In the step of obtaining the crystallized aromatic diol compound, if necessary, a drying step may be carried out. The drying can remove residual solvents, and although the specific drying conditions are not particularly limited, drying can be conducted at a temperature of, for example, 10°C to 100°C, or 10°C to 50°C. Various existing drying technologies can be applied without limitation regarding the specific drying equipment and methods used during the drying process.

Specific examples of the aromatic diol compound include bis(4-hydroxyphenyl)methane, bis(4-hydroxyphenyl) ether, bis(4-hydroxyphenyl)sulfone, bis(4-hydroxyphenyl)sulfoxide, bis(4-hydroxyphenyl)sulfide, bis(4-hydroxyphenyl) ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or mixtures of two or more of these. Preferably, the aromatic diol compound of the monomer for recycled plastic synthesis in this embodiment may be 2,2-bis(4-hydroxyphenyl)propane (bisphenol A).

The purity of the crystallized aromatic diol compound may be 99.94% or more, or 99.95% or more, or 100% or less, or 99.99% or less, or 99.94% to 100%, or 99.95% to 100%, or 99.94% to 99.99%, or 99.95% to 99.99%. The method for measuring the purity of the aromatic diol compound is not particularly limited, and, for example, 1H NMR, ICP-MS analysis, HPLC analysis, UPLC analysis, etc., can be used without limitation. Various known methods, conditions, and equipment for NMR, ICP-MS, HPLC, and UPLC can be applied without restriction.

An example of a method for measuring the purity of the aromatic diol compound involves dissolving the monomer for recycled plastic synthesis in this embodiment at 1% w/v in a methanol (MeOH) solvent under atmospheric pressure at 20 to 30°C, and then analyzing the purity of bisphenol A (BPA) using UPLC (ultra-performance liquid chromatography) with an ACQUITY UPLC® BEH C18 1.7 $\mu$m (2.1*50mm column) on a Waters HPLC system.

As such, in the method for manufacturing monomers for recycled plastic synthesis in this embodiment, the purity of the aromatic diol compound, which is the main recovery target material, is significantly increased to 99.9% or more, minimizing other impurities, thereby enabling excellent physical properties when synthesizing polycarbonate-based resins.

In other words, the monomer for recycled plastic synthesis may further include impurities other than the aromatic diol compound. The impurities refer to all substances excluding the aromatic diol compound, which is the main recovery target material of the present invention, and their specific types are not particularly limited. However, examples include phenol, 4-tert-butylphenol (PTBP), or p-isopropylphenol (IPP).

Meanwhile, the yield of the crystallized aromatic diol compound can be 88% or more, or 90% or more, or 100% or less, or between 88% and 100%, or between 90% and 100%. The method for measuring the yield of the crystallized aromatic diol compound is not particularly limited and can be calculated, for example, using the following formula 1.

[Formula 1]

$$\text{Yield (\%)} = (W1/W0) \times 100$$

In the above formula 1, WO is the mass of the aromatic diol compound obtained when completely decomposed at 100%, and W1 is the actual mass of the aromatic diol compound obtained. In formula 1, the mass of the aromatic diol compound can be measured using various commonly known mass measurement methods without limitation, for example, a scale can be used.

As such, in the method for producing monomers for recycled plastic synthesis according to this embodiment, the yield of the aromatic diol compound, which is the main recovery target substance, is extremely increased to 90% or more, thereby improving the efficiency of the recycling process for polycarbonate-based resins.

Meanwhile, in this embodiment, diethyl carbonate can be obtained as a by-product of the monomer for recycled plastic synthesis. This diethyl carbonate is characterized by being recovered from the polycarbonate-based resin used in the method for producing monomers for recycled plastic synthesis in this embodiment.

That is, as a result of the recovery from the polycarbonate-based resin to obtain the monomer for recycled plastic synthesis in this embodiment, diethyl carbonate is also obtained. Therefore, the addition of new diethyl carbonate from outside, separate from the recovery from the polycarbonate-based resin used in the method for producing monomers for recycled plastic synthesis in this embodiment, is not included in the category of this diethyl carbonate.

Specifically, being recovered from the polycarbonate-based resin means that it is obtained through the depolymerization reaction of the polycarbonate-based resin. This depolymerization reaction can be carried out under acidic, neutral, or basic conditions, and in particular, it can proceed under basic (alkaline) conditions. Specifically, it is preferable that this depolymerization reaction is carried out in an ethanol solvent as described below.

In the monomer for recycled plastic synthesis according to this embodiment, since the main recovery target substance is the aromatic diol compound, the diethyl carbonate can be separately isolated and recovered as a by-product in the method for producing monomers for recycled plastic synthesis in this embodiment.

2. Monomer for synthesizing recycled plastic

According to yet another embodiment of the invention, there can be provided a monomer for synthesizing recycled plastic, comprising an aromatic diol compound obtained by the preparation method of a monomer for synthesizing recycled plastic according to the one embodiment.

That is, the monomer for recycling plastic synthesis of the other embodiments may be obtained by the preparation method of the monomer for recycling plastic synthesis of the one embodiment. The details of the preparation method of the monomer for recycling plastic synthesis of the one embodiment includes all the content described above in the one embodiment.

Specific examples of the aromatic diol compound include bis(4-hydroxyphenyl)methane, bis(4-hydroxyphenyl) ether, bis(4-hydroxyphenyl)sulfone, bis(4-hydroxyphenyl)sulfoxide, bis(4-hydroxyphenyl)sulfide, bis(4-hydroxyphenyl) ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound of the monomer for synthesizing recycled plastics of the one embodiment may be 2,2-bis(4-hydroxyphenyl)propane (bisphenol A).

The aromatic diol compound is characterized by being obtained from the preparation method of a monomer for synthesizing recycled plastic of the one embodiment. That is, the aromatic diol compound is characterized by being recovered from the polycarbonate-based resin used in the recovery of the monomer for synthesizing recycled plastic. Therefore, apart from the recovery from the polycarbonate-based resin in order to prepare the monomer for synthesizing recycled plastics according to the other embodiments, the case where a novel aromatic diol compound is added from the outside is not included in the category of aromatic diol compound of the present disclosure.

Specifically, "being recovered from the polycarbonate-based resin" means being obtained through a depolymerization reaction of the polycarbonate-based resin. The depolymerization reaction can proceed under acidic, neutral or basic conditions, and particularly, the depolymerization reaction can proceed under basic (alkaline) conditions. Particularly, the depolymerization reaction can proceed preferably in the presence of an ethanol solvent, as will be described later.

The monomer for synthesizing recycled plastic in another embodiment can be used as a raw material for manufacturing various recycled plastics (for example, polycarbonate (PC)) to be described later.

The monomer for synthesizing recycled plastic in another embodiment may further include a small amount of other additives and solvents, and the specific types of additives or solvents are not particularly limited, and various substances widely used in the process of recovering aromatic diol compounds by depolymerization of polycarbonate-based resin can be applied without restriction.

3. Recycled Plastic

According to another embodiment of the invention, a recycled plastic comprising a reaction product of the monomer for synthesizing recycled plastic and a comonomer can be provided.

The details of the monomer for synthesizing recycled plastic include all the contents described above in another embodiment.

Examples corresponding to the recycled plastic are not particularly limited, and various plastics synthesized from aromatic diol compounds such as bisphenol A and a carbonate precursor such as dimethyl carbonate, diethyl carbonate, or ethylmethyl carbonate as a monomer can be applied without limitation, and a more specific example may be a polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one carbonate repeating unit obtained by using only one aromatic diol compound and one carbonate precursor, a homopolymer can be synthesized. In addition, when one aromatic diol compound and two or more carbonate precursors are used as the monomer, or two or more aromatic diol compounds and one carbonate precursor are used, or one or more other diols is used in addition to the one aromatic diol compound and the one carbonate precursor to contain two or more carbonates, a copolymer can be synthesized. The homopolymer or copolymer can include all of low-molecular compounds, oligomers, and polymers depending on the molecular weight range.

More specifically, in the recycled plastic containing the reaction product of the monomer for synthesizing the recycled plastic and the comonomer of the one embodiment, a carbonate precursor can be used as the comonomer. Specific examples of the carbonate precursor include phosgene, triphosgene, diphosgene, bromophosgene, dimethyl carbonate, diethyl carbonate, dibutyl carbonate, dicyclohexyl carbonate, diphenyl carbonate, ditolyl carbonate, bis(chlorophenyl)carbonate, m-cresyl carbonate, dinaphthyl carbonate, bis(diphenyl) carbonate or bishaloformate.

Examples of the reaction process of the monomer for synthesizing recycled plastic and the comonomer that synthesizes the polycarbonate-based resin are not particularly limited, and various well-known methods for preparing polycarbonate can be applied without limitation.

However, in one example of the polycarbonate preparation method, a polycarbonate preparation method including the step of polymerizing a containing a monomer for synthesizing recycled plastic and a comonomer can be used. At this time, the polymerization can be carried out by interfacial polymerization, and during interfacial polymerization, polymerization reaction is possible at normal pressure and low temperature, and the molecular weight is easy to control.

The polymerization temperature may be 0°C to 40°C, and the reaction time may be 10 minutes to 5 hours. In addition, the pH during the reaction may be maintained at 9 or more or 11 or more.

The solvent that can be used for the polymerization is not particularly limited as long as it is a solvent used for polymerization of polycarbonate in the art, and as an example, halogenated hydrocarbons such as methylene chloride and chlorobenzene can be used.

Moreover, the polymerization can be carried out in the presence of an acid binder. As the acid binder, an alkali metal hydroxide such as sodium hydroxide or potassium hydroxide, or an amine compound such as pyridine can be used.

Further, in order to control the molecular weight of the polycarbonate during the polymerization, polymerization can be performed in the presence of a molecular weight modifier. An alkylphenol having 1 to 20 carbon atoms may be used as the molecular weight modifier, and specific examples thereof include p-tert-butylphenol, p-cumylphenol, decylphenol, dodecylphenol, tetradecylphenol, hexadecylphenol, octadecylphenol, eicosylphenol, docosylphenol or triacontylphenol. The molecular weight modifier can be added before, during or after the initiation of polymerization. The molecular weight modifier may be used in an amount of 0.01 to 10 parts by weight, or 0.1 to 6 parts by weight, based on 100 parts by weight of the aromatic diol compound, and a desired molecular weight can be obtained within this range.

In addition, in order to promote the polymerization reaction, a reaction accelerator such as a tertiary amine compound, a quaternary ammonium compound, or a quaternary phosphonium compound, including triethylamine, tetra-n-butylammonium bromide, or tetra-n-butylphosphonium bromide can be further used.

4. Molded Product

According to another embodiment of the invention, a molded article comprising the recycled plastic of the other embodiment can be provided. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobiles, electrical and electronic products, communication products, daily necessities, building materials, optical components, exterior materials, and the like.

The molded article may further include one or more additives selected from the group consisting of an antioxidant, a plasticizer, an antistatic agent, a nucleating agent, a flame retardant, a lubricant, an impact enhancer, an optical brightener, an ultraviolet absorber, a pigment and a dye, if necessary, in addition to the recycled plastic of the other embodiments,

An example of the manufacturing method of the molded article may include a step of mixing the recycled plastic of the other embodiment and an additive well using a mixer, extrusion-molding the mixture with an extruder to produce pellets, drying the pellets, and then injecting them with an injection molding machine.

[Advantageous Effects]

According to the present invention, a preparation method of a monomer for synthesizing recycled plastic that contains a high-purity, high-yield aromatic diol compound recovered through recycling by chemical decomposition of a polycarbonate-based resin, a monomer for synthesizing recycled plastic, a recycled plastic and a molded product using the same can be provided.

[DETAILED DESCRIPTION OF THE EMBODIMENTS]

Hereinafter, the present invention will be explained in detail with reference to the following examples. However, these examples are for illustrative purposes only, and the scope of the present invention is not limited thereto.

<EXAMPLE, COMPARATIVE EXAMPLE and REFERENCE EXAMPLE : Preparation of recycled bisphenol A monomer >

Example 1

(1. Pretreatment Step) A solution in which waste polycarbonate (PC) was dissolved in methylene chloride (MC) (containing 18.73 wt% of waste polycarbonate (PC), 78.27 wt% of methylene chloride (MC), and 3 wt% of other impurities) was filtered through a filter with a pore diameter of 3.0 $\mu$m or less to recover the filtrate. At this time, the filtrate contained 18.76 wt% of waste polycarbonate (PC), 78.30 wt% of methylene chloride (MC), and 2.94 wt% of other impurities.

(2. Decomposition Step) The filtrate was introduced into a 3L high-pressure reactor, and ethanol (EtOH), sodium hydroxide (NaOH), and sodium hydrosulfite were added, the atmosphere inside the system was replaced with nitrogen, and the mixture was stirred at 60°C for 6 hours in an inactive state to proceed a PC depolymerization reaction. At this time, for 1 mol of waste polycarbonate (PC), methylene chloride (MC) 17 mol, ethanol (EtOH) 11 mol, and sodium hydroxide (NaOH) 0.25 mol were added to satisfy the molar ratio, and sodium hydrosulfite was added in an amount of 1 wt% relative to the total reaction solution.

(3. Neutralization) The product of the depolymerization reaction was cooled to below 30°C, and then neutralized by adding 10% hydrochloric acid (HCl) and water to achieve a pH of 7. At this time, the neutralized solution contained 10.33 wt% of bisphenol A, 49.53 wt% of methylene chloride (MC), 19.30 wt% of ethanol (EtOH), 13.03 wt% of water, 5.48 wt% of diethyl carbonate (DEC), and 2.33 wt% of other impurities.

(4. Layer Separation) After that, in a state in which the water layer and the methylene chloride (MC) layer were formed, the organic layer located on the bottom side was recovered using a drain device located at the bottom end of the reactor, and the water layer located on the top side was discharged and then discarded. At this time, the methylene chloride (MC) layer contained 11.99 wt% of bisphenol A, 56.76 wt% of methylene chloride (MC), 18.70 wt% of ethanol (EtOH), 4.61 wt% of water, 6.26 wt% of diethyl carbonate (DEC), 0.12 wt% of phenol, 0.37 wt% of 4-tert-butylphenol (PTBP), 0.16 wt% of p-isopropenylphenol (IPP), and 1.03 wt% of other impurities.

(5-1. Distillation - First Distillation under reduced pressure) Thereafter, the recovered methylene chloride (MC) layer was introduced into a first distillation section of a distillation column at a pressure of 0.45 MPa and a temperature of 160°C, and depressurized at a pressure of 0.12 MPa and a temperature of 48~160°C to remove methylene chloride (MC), ethanol (EtOH), and water from the methylene chloride (MC) layer, and the remaining solution was moved to a second distillation section.

(5-2. Distillation - Second Distillation under reduced pressure) Subsequently, in the second distillation section of the distillation column, the remaining solution was pressurized at a pressure of 0.45 MPa and a temperature of 125°C, then depressurized at a pressure of 0.009 MPa and a temperature of 42~160°C to remove diethyl carbonate (DEC) from the remaining solution, and the remaining solution was moved to a third distillation section.

(5-3. Distillation - Third Distillation under reduced pressure) Subsequently, in the third distillation section of the distillation column, the remaining solution was depressurized at a pressure of 0.0005 MPa and a temperature of 155°C, then at a pressure of 0.0003 MPa and a temperature of 42~162°C, to remove diethyl carbonate (DEC) and phenolic impurities from the remaining solution, thereby obtaining a bisphenol A solution.

At this time, the bisphenol A solution contained 90.04 wt% of bisphenol A, 0.52 wt% of diethyl carbonate (DEC), 0.02 wt% of phenol, 1.35 wt% of 4-tert-butylphenol (PTBP), 0.59 wt% of p-isopropenylphenol (IPP), and 7.48 wt% of other impurities.

(6. Melt Crystallization) A fractional melt crystallization process was carried out on 3,200 g of the bisphenol A solution in a falling film dynamic crystallizer (manufactured by Suzler) to obtain recycled bisphenol A monomer. At this time, the recycled bisphenol A monomer obtained in Example 1 contained 99.95 wt% of bisphenol A and 0.05 wt% of bisphenol A dimer.

Specifically, the fractional melt crystallization process proceeds in the following process order.

1) The bisphenol A solution was heated at 185°C for 6-8 hours to melt it.
2) For the molten bisphenol A obtained in 1), a purification cycle consisting of "cooling - sweating - melting" was performed three cycles. The total three purification cycles were carried out at a temperature of 130~180°C for 2-3 hours. Specifically, the purification cycle includes the process of obtaining bisphenol A crystals by cooling the molten bisphenol A, sweating the obtained bisphenol A crystals to obtain purified bisphenol A crystals, and melting the purified bisphenol A crystals.
3) The molten bisphenol A obtained in 2) was recovered and cooled below 157°C in a nitrogen atmosphere to obtain crystallized recycled bisphenol A monomer.

Example 2

Except for changing the number of purification cycles in (6. Melt Crystallization) of Example 1 from three cycles to four cycles, the recycled bisphenol A monomer was produced in the same manner as in Example 1.

Comparative Example 1

(1. Decomposition step) 1 mol of Pretreated waste polycarbonate (PC) was dissolved in 17 mol of methylene chloride(MC), and then added together with 11 mol of ethanol (EtOH) and 0.25 mol of sodium hydroxide (NaOH) to a 3L high-pressure reactor, sodium hydrosulfite as an antioxidant was added in an amount of 0.7 wt% relative to the total solution, the atmosphere inside the system was replaced with nitrogen, and the mixture was stirred at 60°C for 6 hours in an inactive state to proceed a PC depolymerization reaction.

(2. pH adjustment) The product of the depolymerization reaction was cooled to 30°C or less, and then, the bisphenol A was adjusted to have pH 7 by adding 10% hydrochloric acid (HCl) and water to the product.

(3. Layer separation) After that, in a state in which the water layer and the methylene chloride (MC) layer were formed, the organic layer located on the bottom side was recovered using a drain device located at the bottom end of the reactor, and the water layer located on the top side was discharged and then discarded.

(4. Distillation) After that, the recovered methylene chloride (MC) layer was subjected to a low-temperature distillation reducing pressure from 250 mbar and 20~30°C to 30 mbar and 30°C, and thus diethyl carbonate (DEC) as a by-product was separated and recovered.

(5. Purification step-Filtration) After that, the residue from which diethyl carbonate (DEC) was removed was primarily washed using methylene chloride (MC) of twice the weight of PC used at 20~30°C, and vacuum filtered. The filtrate was secondarily washed using water of twice the mass of PC used at a temperature of 50°C.

(6-1. Additional purification step-Redissolving step) After that, bisphenol A was added to 16.6 mol of ethanol and redissolved.

(6-2. Additional purification step - Adsorption step) After that, lignite activated carbon was added as an adsorbent in a ratio of 50 wt% relative to waste polycarbonate, and then purified through adsorption for 3 hours, and then the lignite activated carbon was removed through filtration.

(6-3. Additional purification step - Recrystallization step) After that, 300 mol of water was added to recrystallize bisphenol A, and the obtained slurry was vacuum filtered at 20~30°C to recover bisphenol A (BPA) crystals.

(7. Drying step) After that, it was vacuum dried in a convection oven at 40°C to prepare a recycled bisphenol A monomer in which recycled bisphenol A (BPA) was recovered.

Reference Example 1

Except for conducting the distillation described below (5. Distillation) instead of (5-1. Distillation - First Distillation under reduced pressure), (5-2. Distillation - Second Distillation under reduced pressure), and (5-3. Distillation - Third Distillation under reduced pressure) of Example 1, the recycled bisphenol A monomer was prepared in the same manner as in Example 1.

(5. Distillation) The recovered methylene chloride (MC) layer was subjected to low-temperature distillation by reducing the pressure from 250 mbar, 20 ~ 30 °C to 30 mbar, 30 °C to obtain a bisphenol A solution.

<Experimental Example>

The physical properties of the recycled bisphenol A monomer obtained in the Examples, Comparative Example, and Reference Example were measured by the following methods, and the results are shown in Table 1 below.

For the recycled bisphenol A monomer obtained from the above examples, comparative examples, and reference examples, the physical properties were measured using the following method, and the results are shown in Table 1.

1. Purity

The recycled bisphenol A monomer was dissolved in methanol (MeOH) solvent at 1w% under atmospheric pressure and at 20 ~ 30 °C. Then, the purity of bisphenol A (BPA) was analyzed by ultra performance liquid chromatography (UPLC) on a Waters HPLC system using ACQUITY UPLC®BEH C18 1.7 $\mu$m (2.1*50mm column).

2. Yield

The weight of BPA produced when the polycarbonate used in the reaction is 100% decomposed was measured, and the weight of the obtained BPA was measured, to calculate the yield of BPA as shown in Equation 1 below.

$$[\text{Equation 1}]$$

$$\text{Yield }(\%) = (W1/W0)\text{x}100$$

In Equation 1, WO is the mass of BPA obtained when 100% decomposition occurs, and W1 is the actual mass of BPA obtained. Specifically, when approximately 100 g of polycarbonate is decomposed, the mass of BPA obtained at theoretical 100% decomposition is 89 g. If the actual mass of BPA obtained is 80 g, the yield is (80/89) * 100 = 90%.

[Table 1]

| Measurement result of Experimental Example | | |
|---|---|---|
| Category | Purity (%) | BPA Yield (%) |
| Example 1 | 99.95 | 94 |
| Example 2 | 99.98 | 90 |
| Comparative Example 1 | 99.92 | 82.2 |
| Reference Example 1 | 99.88 | 93.5 |

As shown in Table 1, the recycled bisphenol A monomer obtained in Examples 1 to 2 exhibited a very high purity of 99.95% to 99.98%. Additionally, the recycled bisphenol A monomer obtained in Examples 1 to 2 showed a high BPA yield of 90% to 94%. On the other hand, the recycled bisphenol A monomer obtained in Comparative Example 1 had a lower purity of 99.92% compared to the examples. Furthermore, the recycled bisphenol A monomer obtained in Comparative Example 1 had a BPA yield of 82.2%, which was lower than that of the examples.

**Claims**

1. A preparation method of a monomer for synthesizing recycled plastic, comprising:

a step of recovering an aromatic diol compound obtained from the depolymerization reaction of a polycarbonate-based resin;
a step of melting the aromatic diol compound;
a step of crystallizing the melted aromatic diol compound; and
a step of obtaining the crystallized aromatic diol compound.

2. The preparation method of a monomer for synthesizing recycled plastic according to claim 1, wherein the step of crystallizing the melted aromatic diol compound involves cooling the melted aromatic diol compound to a temperature below 158°C.

3. The preparation method of a monomer for synthesizing recycled plastic according to claim 1, further comprising a step of melting purification of the melted aromatic diol compound at a temperature of 130°C to 180°C for 2 to 6 hours before the step of crystallizing the melted aromatic diol compound.

4. The preparation method of a monomer for synthesizing recycled plastic according to claim 3, wherein the step of melting purification includes:

a step of cooling the melted aromatic diol compound to form crystals of the aromatic diol compound;
a step of partially melting the crystals of the aromatic diol compound to recover purified crystals of the aromatic diol compound; and
a step of melting the purified crystals of the aromatic diol compound.

5. The preparation method of a monomer for synthesizing recycled plastic according to claim 3, wherein the step of melting purification is repeated 2 to 5 cycles.

6. The preparation method of a monomer for synthesizing recycled plastic according to claim 3, wherein the step of melting purification is performed by one-step fractional melting crystallization or multi-step fractional melting crystallization in a falling film dynamic crystallizer.

7. The preparation method of a monomer for synthesizing recycled plastic according to claim 1, wherein the purity of the crystallized aromatic diol compound is 99.94% or more.

8. The preparation method of a monomer for synthesizing recycled plastic according to claim 1, wherein the yield of the crystallized aromatic diol compound is 88% or more.

9. The preparation method of a monomer for synthesizing recycled plastic according to claim 1, wherein the step of recovering the aromatic diol compound obtained from the depolymerization reaction of a polycarbonate-based resin includes:

a step of subjecting the polycarbonate-based resin to a depolymerization reaction;
a step of neutralizing the depolymerization reaction product by adding an acid;
a step of removing the aqueous layer from the aqueous layer and organic solvent layer formed in the neutralization step; and
a step of distilling the organic solvent layer to recover the aromatic diol compound.

10. The preparation method of a monomer for synthesizing recycled plastic according to claim 9, wherein the step of distilling the organic solvent layer to recover the aromatic diol compound includes a multi-stage distillation under reduced pressure step of depressurized distilling the organic solvent layer in three or more stages.

11. The preparation method of a monomer for synthesizing recycled plastic according to claim 10, wherein the multi-stage distillation under reduced pressure step includes:

a first distillation under reduced pressure step of depressurized distilling the organic solvent layer at a pressure of 0.45 MPa or more and a temperature of 160°C or more to a pressure of 0.12 MPa to 0.45 MPa and a temperature of 48°C to 160°C;
a second distillation under reduced pressure step of pressurizing the residual solution from the first distillation under reduced pressure step at a pressure of 0.45 MPa or more and a temperature of 125°C or more, then depressurized distilling at a pressure of 0.009 MPa to 0.45 MPa and a temperature of 42°C to 160°C; and

a third distillation under reduced pressure step of depressurized distilling the residual solution from the second distillation under reduced pressure step at a pressure of 0.0005 MPa or more and a temperature of 155°C or more, then depressurized distilling at a pressure of 0.0003 MPa to 0.0005 MPa and a temperature of 42°C to 87°C.

12. The preparation method of a monomer for synthesizing recycled plastic according to claim 1, wherein the depolymerization reaction of the polycarbonate-based resin is conducted in a solvent containing ethanol.

13. The preparation method of a monomer for synthesizing recycled plastic according to claim 12, wherein the ethanol content is 10 to 15 moles per mole of the polycarbonate-based resin.

14. The preparation method of a monomer for synthesizing recycled plastic according to claim 1, wherein the depolymerization reaction of the polycarbonate-based resin is conducted by reacting a base in a content of 0.5 moles or less per mole of the polycarbonate-based resin.

15. The preparation method of a monomer for synthesizing recycled plastic according to claim 1, further comprising a pretreatment step of passing the polycarbonate-based resin through a filter with a pore diameter of 0.3 $\mu$m or less before the step of recovering the aromatic diol compound obtained by the depolymerization reaction of the polycarbonate-based resin.

16. A monomer for synthesizing recycled plastic, comprising an aromatic diol compound obtained by the preparation method of a monomer for synthesizing recycled plastic according to claim 1

17. A recycled plastic comprising a reaction product of the monomer for synthesizing recycled plastic according to claim 16 and a comonomer.

18. A molded article comprising the recycled plastic according to claim 17.

## INTERNATIONAL SEARCH REPORT

| International application No. |
|---|
| **PCT/KR2025/003154** |

| A. | CLASSIFICATION OF SUBJECT MATTER |
|---|---|

**C07C 37/84**(2006.01)i; **C07C 37/68**(2006.01)i; **C07C 37/74**(2006.01)i; **C07C 37/055**(2006.01)i; **C07C 39/16**(2006.01)i; **C08J 11/24**(2006.01)i; **C08G 64/30**(2006.01)i

According to International Patent Classification (IPC) or to both national classification and IPC

| B. | FIELDS SEARCHED |
|---|---|

Minimum documentation searched (classification system followed by classification symbols)

C07C 37/84(2006.01); C07C 37/52(2006.01); C07C 37/68(2006.01); C07C 37/70(2006.01); C07C 39/16(2006.01); C08G 64/30(2006.01); C08J 11/16(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 해중합(depolymerization), 폴리카보네이트(polycarbonate), 방향족 디올 화합물 (aromatic diol compound), 용융(melting), 결정화(crystallization), 재활용(recycle)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2020-257237 A1 (SABIC GLOBAL TECHNOLOGIES B.V.) 24 December 2020 (2020-12-24) paragraphs [0009], [0020], [0021], [0045], [0050], [0071]; claims 11, 17; table 1 | 1,2,7,8,14,16-18 |
| Y | | 3-6,9-13,15 |
| Y | EP 0475893 A1 (GEBRUDER SULZER AKTIENGESELLSCHAFT) 18 March 1992 (1992-03-18) page 4; claim 1 | 3-6 |
| Y | KR 10-2023-0039100 A (LG CHEM, LTD.) 21 March 2023 (2023-03-21) paragraphs [0063], [0066], [0067], [0077], [0079] | 9-13,15 |
| Y | US 2004-0054238 A1 (BAN, TETSUO et al.) 18 March 2004 (2004-03-18) paragraphs [0002], [0034], [0060] | 10,11 |
| A | US 2016-0168063 A1 (SAUDI BASIC INDUSTRIES CORPORATION) 16 June 2016 (2016-06-16) paragraphs [0016], [0088]; claim 22 | 1-18 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **17 July 2025** | **18 July 2025** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

| International application No. |
| --- |
| **PCT/KR2025/003154** |

| Patent document cited in search report | | | Publication date (day/month/year) | Patent family member(s) | | | Publication date (day/month/year) |
| --- | --- | --- | --- | --- | --- | --- | --- |
| WO | 2020-257237 | A1 | 24 December 2020 | CN | 113993831 | A | 28 January 2022 |
| | | | | CN | 113993831 | B | 02 July 2024 |
| | | | | CN | 114303205 | A | 08 April 2022 |
| | | | | CN | 114730620 | A | 08 July 2022 |
| | | | | EP | 3986850 | A1 | 27 April 2022 |
| | | | | EP | 3987527 | A1 | 27 April 2022 |
| | | | | EP | 3987527 | A4 | 16 August 2023 |
| | | | | EP | 3987528 | A1 | 27 April 2022 |
| | | | | EP | 3987528 | A4 | 16 August 2023 |
| | | | | JP | 2022-537204 | A | 24 August 2022 |
| | | | | JP | 2022-538392 | A | 02 September 2022 |
| | | | | JP | 7253865 | B2 | 07 April 2023 |
| | | | | JP | 7265043 | B2 | 25 April 2023 |
| | | | | KR | 10-2022-0018024 | A | 14 February 2022 |
| | | | | KR | 10-2022-0024436 | A | 03 March 2022 |
| | | | | US | 11923052 | B2 | 05 March 2024 |
| | | | | US | 2020-0402624 | A1 | 24 December 2020 |
| | | | | US | 2020-0402629 | A1 | 24 December 2020 |
| | | | | US | 2024-0203548 | A1 | 20 June 2024 |
| | | | | WO | 2020-257647 | A1 | 24 December 2020 |
| | | | | WO | 2020-257677 | A1 | 24 December 2020 |
| EP | 0475893 | A1 | 18 March 1992 | CN | 1029226 | C | 05 July 1995 |
| | | | | CN | 1060088 | A | 08 April 1992 |
| | | | | EP | 0475893 | B1 | 14 December 1994 |
| | | | | JP | 05-132441 | A | 28 May 1993 |
| | | | | JP | 2949163 | B2 | 13 September 1999 |
| | | | | KR | 10-0216330 | B1 | 16 August 1999 |
| | | | | KR | 10-1992-0006274 | A | 27 April 1992 |
| | | | | US | 5243093 | A | 07 September 1993 |
| | | | | US | 5362900 | A | 08 November 1994 |
| | | | | US | 5475152 | A | 12 December 1995 |
| KR | 10-2023-0039100 | A | 21 March 2023 | CN | 116323773 | A | 23 June 2023 |
| | | | | CN | 116368179 | A | 30 June 2023 |
| | | | | CN | 116368180 | A | 30 June 2023 |
| | | | | CN | 116368181 | A | 30 June 2023 |
| | | | | CN | 116457403 | A | 18 July 2023 |
| | | | | CN | 116615492 | A | 18 August 2023 |
| | | | | EP | 4209528 | A1 | 12 July 2023 |
| | | | | EP | 4209528 | A4 | 01 May 2024 |
| | | | | EP | 4209529 | A1 | 12 July 2023 |
| | | | | EP | 4209529 | A4 | 10 July 2024 |
| | | | | EP | 4209530 | A1 | 12 July 2023 |
| | | | | EP | 4209530 | A4 | 24 April 2024 |
| | | | | EP | 4209531 | A1 | 12 July 2023 |
| | | | | EP | 4209531 | A4 | 01 May 2024 |
| | | | | EP | 4212572 | A1 | 19 July 2023 |
| | | | | EP | 4212572 | A4 | 19 June 2024 |
| | | | | EP | 4230679 | A1 | 23 August 2023 |
| | | | | EP | 4230679 | A4 | 17 July 2024 |
| | | | | JP | 2023-545936 | A | 01 November 2023 |

Form PCT/ISA/210 (patent family annex) (July 2022)

## INTERNATIONAL SEARCH REPORT
### Information on patent family members

International application No.

**PCT/KR2025/003154**

| Patent document cited in search report | | | Publication date (day/month/year) | Patent family member(s) | | | Publication date (day/month/year) |
|---|---|---|---|---|---|---|---|
| | | | | JP | 2023-545937 | A | 01 November 2023 |
| | | | | JP | 2023-545938 | A | 01 November 2023 |
| | | | | JP | 2023-545953 | A | 01 November 2023 |
| | | | | JP | 2023-545954 | A | 01 November 2023 |
| | | | | JP | 2023-550948 | A | 06 December 2023 |
| | | | | JP | 7584637 | B2 | 15 November 2024 |
| | | | | JP | 7585473 | B2 | 18 November 2024 |
| | | | | JP | 7607757 | B2 | 27 December 2024 |
| | | | | JP | 7621482 | B2 | 24 January 2025 |
| | | | | JP | 7665741 | B2 | 21 April 2025 |
| | | | | KR | 10-2023-0039099 | A | 21 March 2023 |
| | | | | KR | 10-2023-0039101 | A | 21 March 2023 |
| | | | | KR | 10-2023-0039102 | A | 21 March 2023 |
| | | | | KR | 10-2023-0045976 | A | 05 April 2023 |
| | | | | KR | 10-2023-0052755 | A | 20 April 2023 |
| | | | | US | 2023-0374251 | A1 | 23 November 2023 |
| | | | | US | 2023-0382837 | A1 | 30 November 2023 |
| | | | | US | 2023-0383089 | A1 | 30 November 2023 |
| | | | | US | 2023-0383090 | A1 | 30 November 2023 |
| | | | | US | 2023-0391702 | A1 | 07 December 2023 |
| | | | | US | 2024-0002627 | A1 | 04 January 2024 |
| | | | | WO | 2023-038266 | A1 | 16 March 2023 |
| | | | | WO | 2023-038267 | A1 | 16 March 2023 |
| | | | | WO | 2023-038268 | A1 | 16 March 2023 |
| | | | | WO | 2023-038269 | A1 | 16 March 2023 |
| | | | | WO | 2023-038270 | A1 | 16 March 2023 |
| | | | | WO | 2023-038271 | A1 | 16 March 2023 |
| US | 2004-0054238 | A1 | 18 March 2004 | CN | 1481347 | A | 10 March 2004 |
| | | | | CN | 1481347 | C | 10 March 2004 |
| | | | | EP | 1439158 | A1 | 21 July 2004 |
| | | | | EP | 1439158 | A4 | 05 January 2005 |
| | | | | KR | 10-2004-0055726 | A | 26 June 2004 |
| | | | | WO | 03-035592 | A1 | 01 May 2003 |
| US | 2016-0168063 | A1 | 16 June 2016 | CN | 105658712 | A | 08 June 2016 |
| | | | | EP | 3058019 | A1 | 24 August 2016 |
| | | | | JP | 2016-533416 | A | 27 October 2016 |
| | | | | US | 2015-0105531 | A1 | 16 April 2015 |
| | | | | US | 9328046 | B2 | 03 May 2016 |
| | | | | WO | 2015-057682 | A1 | 23 April 2015 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240151994